# EUROPÄISCHE PATENTSCHRIFT

(11) **EP 3 278 727 B1**
(45) Veröffentlichungstag und Bekanntmachung des Hinweises auf die Patenterteilung: **30.09.2020**
(21) Anmeldenummer: 17170364.8
(22) Anmeldetag: 10.05.2017
(51) Int. Cl.: A61B 5/107

(54) **MESSEINRICHTUNG ZUR BESTIMMUNG DER KÖRPERGRÖSSE EINES PATIENTEN**
DEVICE TO MEASURE BODY HEIGHT OF A PATIENT
DISPOSITIF DE MESURE DE MESSURE DE TAILLE D'UN PATIENT

(30) Priorität: 04.08.2016 DE 102016009606
(43) Veröffentlichungstag der Anmeldung: 07.02.2018
(73) Patentinhaber: seca ag, 4153 Reinach BL 1 (CH)
(72) Erfinder: Huß, Kirsten, 22926 Ahrensburg (DE)
(74) Vertreter: Klickow & Wetzel PartGmbB

(56) Entgegenhaltungen:
- DE-B3-102012 220 412
- US-A1- 2003 159 300

## Beschreibung

Die Erfindung betrifft eine Messeinrichtung zur Bestimmung der Körpergröße eines Patienten, mit einer an einer Standsäule befestigten berührungslosen Messanordnung, wobei die Messanordnung eingerichtet ist, ein Messsignal in Richtung des Kopfes eines unter der Messanordnung stehenden Patienten auszusenden, das reflektierte Messsignal aufzufangen und aus dem aufgefangenen Messsignal die Entfernung des Kopfes des Patienten von der Messanordnung zu bestimmen, und mit einer Steuerung die eingerichtet ist, aus der so bestimmten Entfernung und der bekannten Position der Messanordnung die Körpergröße des Patienten zu bestimmen, wobei die Messeinrichtung weiterhin einen entlang der Standsäule verfahrbaren Kopfanschlag aufweist.

Eine solche Messeinrichtung ist bereits bekannt. Gegenüber einer klassischen Messeinrichtung für die Körpergröße eines Patienten, bei welcher ein Kopfanschlag an einer Standsäule entlang eines Maßstabes verschoben wird, bis er auf dem Kopf des Patienten aufliegt, und dann die Position des Kopfanschlags an dem Maßstab optisch oder elektronisch abgelesen wird, weist eine gattungsgemäße Messeinrichtung den Vorteil auf, dass auf eine Berührung des Patienten verzichtet werden kann. Eine solche Berührung wird von Patienten zum Teil als unangenehm empfunden und kann im Einzelfall Schmerzen oder Angstgefühle verursachen.

Bei der berührungslosen Messung werden in der Regel mehrere Messungen in schneller Folge durchgeführt, um dann den Mittelwert der ermittelten Messwerte als Endergebnis zu verwenden. Dadurch können Messungenauigkeiten reduziert werden.

Es ist hierfür erforderlich, dass der Patient einige Sekunden möglichst bewegungslos unter der Messanordnung steht. In der Praxis hat sich jedoch gezeigt, dass nicht alle Patienten in der Lage sind, diese Anforderung zu erfüllen. Insbesondere Kinder neigen dazu, sich trotz entsprechender Aufforderung unruhig zu bewegen, dadurch wird eine genaue Messung der Körpergröße erschwert.

Aus der US 2003/159300 A1 ist bereits eine Messeinrichtung zur Bestimmung der Körpergröße eines Patienten bekannt, bei der an einer Standsäule eine Messanordnung zur Durchführung einer berührungslosen Messung angeordnet ist. Die Messanordnung sendet ein Messsignal in Richtung eines Kopfes, eines unter der Messanordnung stehenden Patienten und empfängt ein vom Patienten zurückgeworfenes Reflektionssignal. Basierend auf dem reflektierten Messisgnal wird die Entfernung zwischen dem Kopf des Patienten und der Messanordnung bestimmt.

Eine ähnliche Messanordnung unter Verwendung eines Ultraschallsensors wird in der DE 10 2012 220412 B3 beschrieben.

Es besteht daher die Aufgabe der Erfindung darin, eine Messeinrichtung und ein entsprechendes Messverfahren der einleitend genannten Art bereitzustellen, die hinsichtlich der beschriebenen Problematik verbessert sind.

Diese Aufgabe wird erfindungsgemäß gelöst, dadurch dass dem Kopfanschlag ein Sensor zur Bestimmung der Schwenkposition des Kopfanschlages zugeordnet ist, und dass die Messanordnung und/oder die Steuerung eingerichtet ist, anhand der so bestimmten Schwenkposition des Kopfanschlages zu erkennen, ob eine berührungslose Messung oder eine Messung mittels des Kopfanschlages durchgeführt wird, wobei die Steuerung eingerichtet ist, eine Dicke des Kopfanschlages bei der Bestimmung der Körpergröße des Patienten zu berücksichtigen.

Es ist dadurch möglich, abhängig von der Befähigung des Patienten, sich während der Messung nicht zu bewegen, entweder eine berührungslose Messung oder eine Messung unter Nutzung des Kopfanschlags durchzuführen. Somit kann mittels der erfindungsgemäßen Messeinrichtung praktisch immer eine erfolgreiche Messung der Körpergröße des Patienten durchgeführt werden, ohne auf die Vorzüge der berührungslosen Messung ganz verzichten zu müssen,

Die Bestimmung der Körpergröße mittels des Kopfanschlags kann elektronisch oder optisch unter Verwendung eines Maßstabs erfolgen, so wie es von klassischen Messeinrichtungen bekannt ist. Für eine automatische Dokumentation der Messwerte müssten hierfür allerdings zwei komplett unabhängige Messanordnungen in der Messeinrichtung vorgehalten werden, was zu unerwünscht hohen Herstellkosten führen würde.

Gemäß einer Weiterbildung der Erfindung ist daher der Kopfanschlag eingerichtet, ein von der Messanordnung ausgesendetes Messsignal zu reflektieren, und die Steuerung ist eingerichtet, eine Dicke des Kopfanschlags bei der Bestimmung der Körpergröße des Patienten zu berücksichtigen. Die Bestimmung der Körpergröße kann daher sowohl bei berührungsloser Messung als auch bei Messung mittels Kopfanschlag mit der berührungslosen Messanordnung durchgeführt werden. Dazu ist die Dicke des Kopfabschlags in der Steuerung gespeichert und wird bei Messung mittels Kopfanschlag von dem ermittelten Messwert subtrahiert.

Damit der Kopfanschlag bei berührungsloser Messung kein die Messung störendes Reflexionssignal liefert, ist der Kopfanschlag bei einer vorteilhaften Ausführung der Erfindung derart verschwenkbar an der Standsäule angeordnet, dass er in einer ersten Schwenkposition ein von der Messanordnung ausgesendetes Messsignal reffektiert, und dass er in einer zweiten Schwenkposition ein von der Messanordnung ausgesendetes Messsignal nicht oder nur in einem sehr geringen Ausmaß reflektiert.

Im Sinne der Erfindung ist das Merkmal, dass der Kopfanschlag in der zweiten Schwenkposition ein von der Messanordnung ausgesendetes Messsignal nicht oder nur in einem sehr geringen Ausmaß reflektiert, so zu verstehen, dass eine Reflexion in Richtung der Messanordnung unterbleibt. Dabei kann eine Reflexion in andere Richtungen ohne weiteres in Kauf genommen werden.

Dazu kann der Kopfanschlag an einer Außenfläche, welche in der zweiten Schwenkposition in Richtung der Messanordnung ausgerichtet ist, reflexionsmindernd ausgestaltet sein. Dazu kann diese Fläche beispielsweise angeschrägt sein oder eine reflexionsmindernde Beschichtung oder Oberflächenstrukturierung aufweisen.

Um die Verfahrbarkeit und die Verschwenkbarkeit des Kopfanschlags unabhängig voneinander zu ermöglichen, ist in einer Ausführung der Erfindung der Kopfanschlag an einem einen entlang der Standsäule verfahrbaren Schlitten verschwenkbar angeordnet.

Dabei ist der Kopfanschlag vorzugsweise in der ersten Schwenkposition arretierbar ausgeführt. Die Arretierung kann beispielsweise mittels eines Rastanschlags oder mittels eines magnetischen Anschlags erfolgen. Dabei ist der Kopfanschlag vorteilhafterweise in der ersten Schwenkposition waagerecht ausgerichtet. In der zweiten Schwenkposition kann hingegen der Kopfanschlag senkrecht nach unten hängen oder nach oben geklappt sein und sich somit in einer eigenstabilen Position befinden, so dass auf eine Arretierung in dieser Schwenkposition verzichtet werden kann.
Es ist weiterhin vorteilhaft, wenn der Kopfanschlag in einer Verfahrposition entlang der Standsäule arretierbar ausgeführt ist. Dadurch wird ein die Messung störendes Verrutschen des Kopfanschlags durch Bewegungen des Patienten verhindert. Die Arretierung kann hier beispielsweise mittels einer Rastung oder einer selbstgreifenden Bremse erfolgen.

In einer möglichen Ausgestaltung der Erfindung können die Messanordnung und/oder die Steuerung eingerichtet sein, anhand des reflektierten Messsignals zu erkennen, ob eine berührungslose Messung oder eine Messung mittels des Kopfanschlages durchgeführt wird.

Dazu kann beispielsweise die Messanordnung so ausgeführt sein, dass eine Entfernungsmessung an mehreren räumlich getrennten Punkten erfolgt. Weichen die Messwerte an diesen Punkten voneinander ab, so kann davon ausgegangen werden, dass eine berührungslose Messung durchgeführt wird. Sind hingegen die Messwerte an allen Punkten im Wesentlichen gleich, so kann davon ausgegangen werden, dass die Messung mittels des waagerecht angeordneten Kopfanschlags erfolgt.

Alternativ oder zusätzlich können Schärfe, Intensität oder Amplitudenverlauf des reflektierten Messsignals ausgewertet werden. So ergibt sich z.B. bei der Reflexion an einem mit Haaren bedeckten Kopf ein weniger scharfes und schwächeres reflektiertes Messsignal als bei Reflexion an einem kompakten und glatten Kopfanschlag. Ebenso kann sich eine Stufe im Amplitudenverlauf des reflektierten Messsignals ergeben, wenn ein Teil des Messsignals an der Oberfläche des Kopfanschlags reflektiert wird, und ein weiterer Teil des Messsignals an der weiter entfernten Kopfoberfläche.

In einer alternativen Ausgestaltung der Erfindung ist dem Kopfanschlag ein Sensor zur Bestimmung der Schwenkposition des Kopfanschlages zugeordnet, und dass die Messanordnung und/oder die Steuerung eingerichtet ist, anhand der so bestimmten Schwenkposition des Kopfanschlages zu erkennen, ob eine berührungslose Messung oder eine Messung mittels des Kopfanschlages durchgeführt wird. In einer weiteren alternativen Ausgestaltung ist der Kopfanschlag derart ausgebildet, dass er ein spezifisches Echo derart generiert, dass eine Erkennung erfolgen kann.
Als Sensor kann hier beispielsweise ein Näherungsschalter oder ein Neigungssensor Anwendung finden.

Gemäß einer weiteren alternativen Ausgestaltung der Erfindung umfasst die Messeinrichtung eine Eingabevorrichtung, und/oder die Messanordnung und/oder die Steuerung ist eingerichtet, anhand einer Eingabe durch einen Benutzer zu entscheiden, ob eine berührungslose Messung oder eine Messung mittels des Kopfanschlages durchgeführt werden soll.

Als Eingabevorrichtung kann beispielsweise ein an der Standsäule befestigtes Touch-Panel Verwendung finden, über welches auch weitere Funktionen der medizinischen Messeinrichtung gesteuert werden können.

Die Aufgabe wird weiterhin gelöst durch ein Messverfahren zur Bestimmung der Körpergröße eines Patienten, mit den Schritten:
Positionieren eines Patienten unter einer berührungslosen Messanordnung, wahlweise
Positionieren eines Kopfanschlags an der Oberseite des Kopfes des Patienten im Erfassungsbereich der Messanordnung, oder
Entfernen des Kopfanschlags aus dem Erfassungsbereich der Messanordnung, und
Bestimmen der Körpergröße des Patienten mittels der Messanordnung,
wobei die Dicke des Kopfanschlags bei der Bestimmung der Körpergröße des Patienten berücksichtigt wird, wenn der Kopfanschlag im Erfassungsbereich der Messanordnung positioniert ist.

Die Erfindung wird nachfolgend anhand einiger exemplarischer Ausführungsbeispiele und Zeichnungen erläutert. Es zeigen:
- Fig. 1:: Eine Messeinrichtung in einer ersten Konfiguration,
- Fig. 2:: eine Messeinrichtung in einer zweiten Konfiguration,
- Fig. 3:: eine Messanordnung einer Messeinrichtung,
- Fig. 4:: eine prinzipielle Darstellung der Signalauswertung in einer Messeinrichtung,
- Fig. 5:: einen Kopfanschlag einer Messeinrichtung.

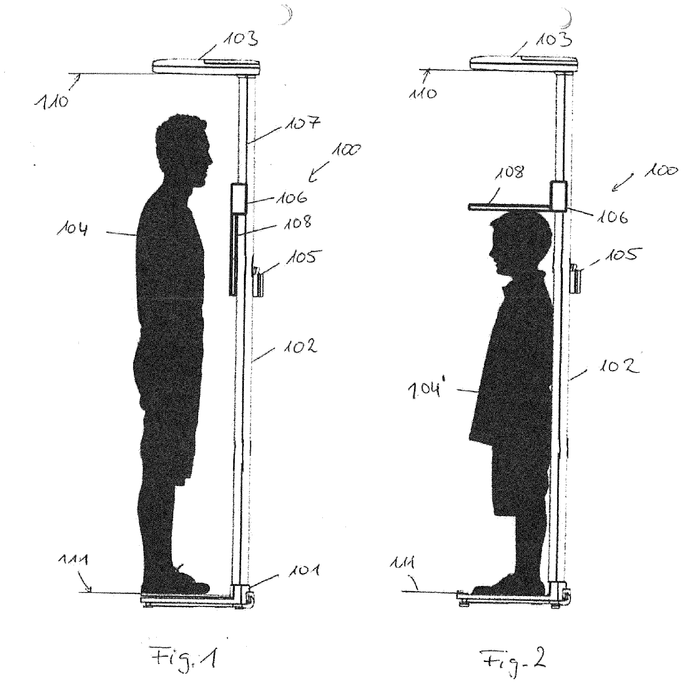

Figur 1 zeigt eine Messeinrichtung 100, mit einem Standfuß 101, einer Standsäule 102 und einer Messanordnung 103. In dem Standfuß 101 ist im dargestellten Beispiel eine Waage integriert, welche automatisch das Körpergewicht eines in der Messeinrichtung 100 stehenden Patienten 104 bestimmt.

Die Messanordnung 103 ist dazu bestimmt, die Körpergröße des Patienten 104 zu bestimmen.

An der Standsäule 102 ist eine Steuerung 105 angeordnet, welche mit einer Anzeige und eine Eingabevorrichtung ausgestattet ist. Zusätzlich kann die Steuerung 105 eine nicht dargestellte drahtlose Kommunikationsschnittstelle aufweisen, mittels welcher Patienteninformationen und Messergebnisse mit einem entfernten Datenverarbeitungssystem, beispielsweise einem Patientendatenmanagementsystem (PDMS), ausgetauscht werden können.

An der Standsäule 102 ist weiterhin ein Schlitten 106 verfahrbar angeordnet. Der Schlitten läuft dazu in einer entlang der Standsäule 102 verlaufenden Führungsnut 107. An dem Schlitten 106 ist ein Kopfanschlag 108 verschwenkbar angeordnet.

Der Patient 104 gemäß Figur 1 ist erwachsen und somit in der Lage, hinreichend still zu stehen, um eine berührungslose Messung der Körpergröße mittels der Messanordnung 103 zu ermöglichen. Daher ist der Kopfanschlag 108 in eine senkrechte, nach unten gerichtete Position geschwenkt, in welcher er die Messung durch die Messanordnung 103 wenig behindert.

Die Bestimmung der Körpergröße des Patienten 104 wird durch ein von der Steuerung 105 ausgelöstes Anforderungssignal gestartet. Dies kann durch manuelle Eingabe über die Eingabevorrichtung, über die drahtlose Kommunikationsschnittstelle, oder automatisch beispielsweise anhand des von der Waage übermittelten Gewichtsmesswerts ausgelöst werden.

Zur Bestimmung der Körpergröße des Patienten 104 sendet die Messanordnung 103 ein Messsignal in Richtung des Patienten 104 aus. Das Messsignal wird am Kopf des

Patienten 104 reflektiert und von der Messanordnung 103 wieder aufgefangen. Aus dem reflektierten Messsignal bestimmt die Messanordnung 103 die Entfernung der höchsten Stelle des Kopfes des Patienten 104 von einer oberen Referenzebene 110 der Messanordnung 103.

Die so bestimmte Entfernung wird an die Steuerung 105 übertragen. In der Steuerung ist die Entfernung der oberen Referenzebene 110 der Messanordnung von einer unteren Referenzebene 111 der Messeinrichtung 100 gespeichert. Die Steuerung berechnet aus der Entfernung der oberen Referenzebene 110 von der höchsten Stelle des Kopfes des Patienten 104 einerseits und der Entfernung der oberen Referenzebene 110 von der unteren Referenzebene 111 andererseits die Körpergröße des Patienten 104. Die so ermittelte Körpergröße und das mittels der Waage ermittelte Körpergewicht des Patienten werden über die Anzeige der Steuerung 105 ausgegeben und ggf. mittels der drahtlosen Kommunikationsschnittstelle weitergeleitet.

In Figur 2 ist die gleiche medizinische Messeinrichtung 100 dargestellt. Zur Vermeidung von Wiederholungen wird hier auf die erneute Beschreibung sich entsprechender Komponenten weitestgehend verzichtet.

In der in Figur 2 dargestellten Konfiguration sollen Körpergewicht und Körpergröße eines Patienten 104' bestimmt werden. Der Patient 104' ist ein Kind und hat daher Schwierigkeiten, ausreichend lang stillzustehen, um eine berührungslose Messung durch die Messanordnung 103 zu ermöglichen.

Um trotzdem einen verlässlichen Messwert für die Körpergröße des Patienten 104' zu erhalten, wird der Schlitten 106 entlang der Standsäule 102 über den Kopf des Patienten 104' verfahren, danach wird der Kopfanschlag 108 in eine waagerechte Schwenkposition verschwenkt, in welcher der Kopfanschlag 108 das von der Messanordnung 103 ausgesendete Messsignal reflektiert, und in dieser Schwenkposition arretiert. Dann wird der Schlitten 106 so weit nach unten verfahren, bis der Kopfanschlag 108 auf dem Kopf des Patienten 104' aufliegt, und wiederum in dieser Position arretiert.

Während der beschriebenen Ausrichtung des Kopfanschlags 108 ist es ratsam, dass sich der Patient 104' mit geradem Rücken an die Standsäule 102 stellt.

Es wird dann wie schon zu Figur 1 beschrieben, die Bestimmung der Körpergröße des Patienten 104' ausgeführt. Dabei wird das von der Messanordnung 103 ausgesendete Messsignal jedoch nicht vom Kopf des Patienten 104' reflektiert, sondern von der Oberseite des Kopfanschlags 108, welche um die Dicke des Kopfanschlags 108 in Richtung der oberen Referenzebene 110 versetzt ist. Die Messanordnung ermittelt somit eine geringere Entfernung als der Körpergröße des Patienten 104' entsprechen würde.

Der Steuerung 105 liegt jedoch aufgrund einer manuellen Eingabe über die Eingabevorrichtung, der Auswertung des reflektierten Messsignals, oder eines dem Kopfanschlag 108 zugeordneten Sensors die Information vor, dass die Messung der Körpergröße unter Einsatz des Kopfanschlags 108 durchgeführt worden ist. Die Steuerung 105 kann daher die Dicke des Kopfanschlags 108, die bekannt und in der Steuerung 105 gespeichert ist, von der ermittelten scheinbaren Körpergröße abziehen und so die korrekte Körpergröße des Patienten 104' bestimmen und ausgeben.

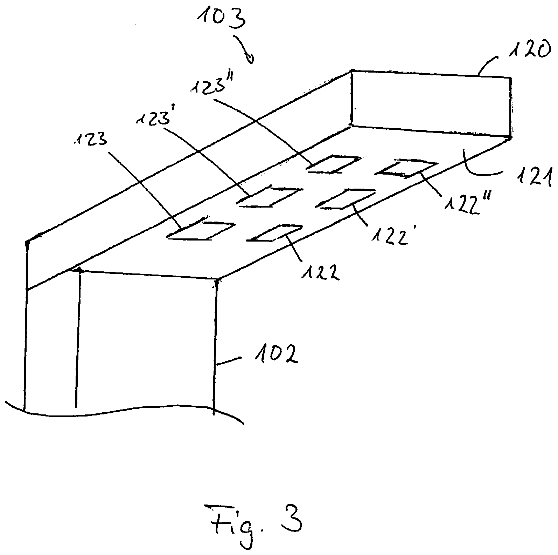

Figur 3 zeigt die Messanordnung 103 in einer vereinfachten perspektivischen Darstellung. Die Messanordnung 103 weist ein am oberen Ende der Standsäule 102 befestigtes Gehäuse 120 auf. An der Unterseite 121 des Gehäuses 120 sind mehrere Sender 122, 122', 122" und Empfänger 123, 123', 123" paarweise angeordnet, wobei jeweils einem der Sender 122, 122', 122" einer der Empfänger 123, 123', 123" zugeordnet ist. Anstelle von drei Sender-Empfänger-Paaren 122, 123, 122', 123', 122", 123" kann auch eine kleinere oder größere Anzahl von Sender-Empfänger-Paaren verwendet werden, es sollten aber in jedem Fall genug Sender-Empfänger-Paare vorhanden sein, um die Kontur des Kopfes des Patienten 104 abzutasten und die höchste Stelle zur Bestimmung der tatsächlichen Körpergröße zu bestimmen.

Bei den Sendern 122, 122', 122" kann es sich um optische Sender, z.B. Laserdioden, handeln, die ein optisches Messsignal aussenden. Dass Messsignal kann frequenz,-amplituden- oder pulsmoduliert sein. In diesem Fall sind die Empfänger 123, 123', 123" als optische Empfänger ausgeführt, z.B. als Fotodiode, Fototransistor oder lichtabhängiger Widerstand (LDR). Für eine optische Abstandsmessung kann die Intensität oder die Intensitätsverteilung des von den Empfängern 123, 123', 123" aufgefangenen reflektierten Messsignals ausgewertet werden, ebenso kann die Laufzeit des Messsignals vom jeweiligen Sender 122, 122', 122" bis zum jeweiligen Empfänger 123, 123', 123" ausgewertet werden. Bekannte Messverfahren sind z.B. die Lasertriangulation oder die time-of-flight (TOF) Messung.

Vorzugsweise handelt es sich bei den Sendern 122, 122', 122" um Ultraschallsender und bei den Empfängern 123, 123', 123" um Ultraschallempfänger. Bei einer Entfernungsmessung mittels Ultraschall wird bevorzugt die Laufzeit des Messsignals vom jeweiligen Sender 122, 122', 122" bis zum jeweiligen Empfänger 123, 123', 123" ausgewertet. Dazu kann das Messsignal ebenfalls frequenz-, amplituden- oder pulsmoduliert sein.

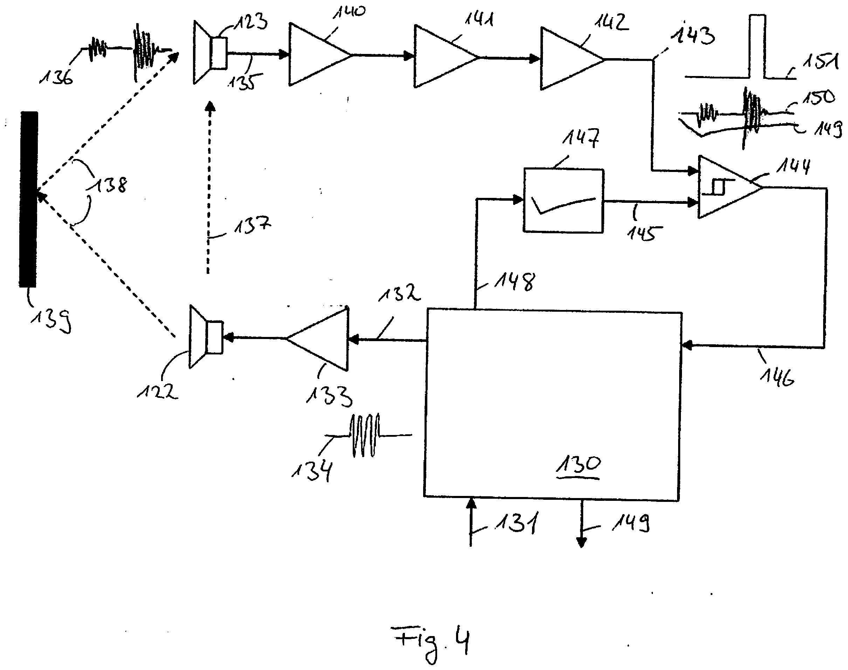

Der prinzipielle Aufbau und der Ablauf der Signalauswertung zur berührungslosen Abstandsmessung werden anhand der Figur 4 beschrieben, wobei exemplarisch von einer Messung mittels pulsmodulierten Ultraschallsignalen ausgegangen wird.

Die Abstandsmessung wird von einem Mikrocontroller 130 gesteuert. Sobald der Mikrokontroller 130 ein Anforderungssignal 131 von der Steuerung 105 (in Fig. 4 nicht dargestellt) erhält, sendet der Mikrokontroller ein Ansteuerungssignal 132 an einen Signalverstärker 133, welcher das Ansteuerungssignal 132 verstärkt und an einen als Ultraschallsender ausgeführten Sender 122 ausgibt.
Die Signalform des Ansteuerungssignals 132 ist mit der Linie 134 angedeutet, es handelt sich im Wesentlichen um einen Rechteckpuls mit einer konstanten Grundfrequenz. Das Ansteuerungssignal 132 kann durch den Mikrokontroller 132 beispielsweise mittels direkter digitaler Synthese (DDS) erzeugt werden.

Der Sender erzeugt aus dem verstärkten Ansteuerungssignal 132 ein Messsignal in Form eines Ultraschallpulses. Das Messsignal wird von einem als Ultraschallempfänger ausgeführten Empfänger 123 aufgefangen und in ein Empfangssignal 135 umgesetzt.

Die Signalform des Empfangssignals 135 ist mit der Linie 136 angedeutet. Es ist zu erkennen, dass das Empfangssignal zwei aufeinander folgende Rechteckpulse aufweist. Dies liegt daran, dass das Messsignal auf zwei möglichen Signalwegen von dem Sender 122 zu dem Empfänger 123 gelangen kann.

Ein direkter, unerwünschter Signalweg führt direkt vom Sender 122 zum Empfänger 123 und entsteht durch mechanisches und/oder elektrisches Übersprechen. Die Übertragungsintensität entlang des Übertragungswegs 137 wird durch mechanische und/oder elektrische Abschirmmaßnahmen so weit wie möglich reduziert.

Ein zweiter, erwünschter Signalweg 138 führt vom Sender 122 zum Messobjekt 139 und von dort zum Empfänger 123. Durch den langen Übertragungsweg und eine unvollständige Reflexion am Messobjekt 139 weist der Übertragungsweg 138 ebenfalls eine geringe Übertragungsintensität auf.

Das Empfangssignal 135 wird in der Folge durch einen ersten Verstärker 140, einen Bandpassfilter 141 sowie einen zweiten Verstärker 142 zu einem Vergleichssignal verarbeitet. Der Bandpassfilter 141 kann z.B. dazu verwendet werden, Signale benachbarter Sender-Empfänger-Paare 122', 123', 122", 123" auszufiltern. Dazu werden die jeweiligen Sender-Empfänger-Paare 122, 123, 122', 123', 122", 123" vorzugs- weise mit unterschiedlichen Grundfrequenzen betrieben.

Das Vergleichssignal 143 wird dann in einem Komparator 144 mit einem Schwellwertsignal 145 verglichen, um ein Detektionssignal 146 zu erhalten. In den Komparator ist vorzugsweise ein (nicht dargestellter) an die Grundfrequenz des Messsignals angepasster Monovibrator integriert, so dass als Detektionssignal 146 ein einfacher Rechteckpuls erzeugt wird, solange die Amplitude des Vergleichssignals 143 die Amplitude des Schwellwertsignals 145 überschreitet.

Das Schwellwertsignal wird in einem Rampengenerator 147 erzeugt, welcher durch den Mikrokontroller 130 gleichzeitig mit der Erzeugung des Ansteuerungssignals 132 durch ein Triggersignal 148 gestartet wird.

Ein möglicher zeitlicher Verlauf des Schwellwertsignals 145, des Vergleichsignals 143 und des Detektionssignals ist mit den Linien 149, 150 und 151 angedeutet.

Linie 150 zeigt den Verlauf des Vergleichssignals 143 mit einem ersten Puls, welcher aus dem unerwünschten Übersprechen zwischen Sender 122 und Empfänger 123 resultiert, und einem zweiten Puls, welcher aus dem am Messobjekt 139 reflektierten Messsignal resultiert. Im dargestellten Beispiel weist der zweite Puls eine etwas höhere Amplitude auf als der erste Puls, da das Übersprechen durch entsprechende Maßnahmen stark reduziert ist. Um den unerwünschten ersten Puls auszublenden, weist das mit Linie 149 dargestellte Schwellwertsignal 145 kurz nach dem Erzeugen des Ansteuerungssignals 132 eine hohe Amplitude auf, so dass die Amplitude des Vergleichssignals 143 während des ersten Pulses nicht ausreicht, um den Komparator 144 auszulösen.

Es ist erkennbar, dass die Amplitude des Schwellwertsignals 145 mit der Zeit abfällt, so dass zum Zeitpunkt des Eintreffens des zweiten Pulses durch das reflektierte Messsignal die Amplitude des Vergleichssignals 143 ausreicht, um den Komparator auszulösen. Für die Dauer des zweiten Pulses weist daher das Detektionssignal einen Rechteckimpuls auf, der in der Linie 151 angedeutet ist.

Die Amplitude des reflektierten Messsignals nimmt mit dem Quadrat der Entfernung des Messobjekts 139 von dem Sender-Empfänger-Paar 122, 123 ab. Um auch bei größeren Entfernungen eine sichere Erkennung des reflektierten Messsignals zu gewährleisten, fällt die Amplitude des Schwellwertsignals 145 in einem ähnlichen Maß ab, beispielsweise nach einer Funktion S(t)=S0/t².

Das Detektionssignal 146 wird in den Mikrokontroller 130 zurückgeleitet. Dieser bestimmt aus dem Zeitabstand zwischen der Erzeugung des Ansteuerungssignals 132 und dem Empfang des Detektionssignals 146 unter Berücksichtigung bekannter Signallaufzeiten in der Signalaufbereitung die Entfernung des Messobjekts 139 vom Sender-Empfänger-Paar 122, 123. Die so ermittelte Entfernung wird als Ergebnissignal 149 an die Steuerung 105 ausgegeben.

Die Schaltungskomponenten 130, 133, 140, 141, 142, 144 und 147 sind zur Reduzierung der Signallaufzeiten bevorzugt nahe bei den Sendern-Empfänger-Paaren 122, 123, 122', 123', 122", 123" angeordnet, besonders bevorzugt innerhalb des Gehäuses 120.

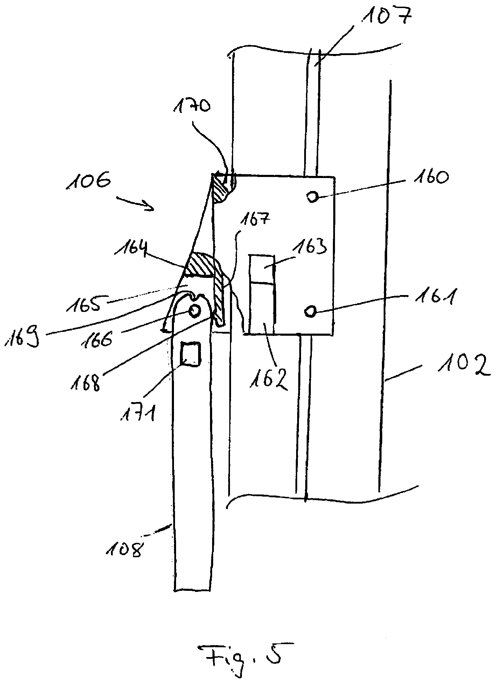

In Figur 5 ist ein möglicher Aufbau eines Kopfanschlags einer medizinischen Messeinrichtung dargestellt. An der Standsäule 102 ist ein Schlitten 106 angeordnet, der die Standsäule U-förmig halb umgreift. An den Schenkeln des Schlittens 106 sind Führungsstifte 160, 161 angebracht, welche in der Führungsnut 107 der Standsäule 102 laufen und ein Verkippen des Schlittens 106 verhindern.

An einer Seite des Schlittens 106 ist eine Federzunge 162 ausgeschnitten, welche gegen die Standsäule 102 drückt und damit den Schlitten 106 arretiert. Am oberen Ende der Federzunge 162 ist ein Hebel 163 angeformt, mittels dessen die Federzunge 162 zum Lösen der Arretierung elastisch nach außen gebogen werden kann.

Auf der dem Patienten zugewandten Seite weist der Schlitten 106 einen sägezahnförmigen Vorsprung 164 auf, welcher im unteren Bereich einen mittigen Freiraum 165 zur Aufnahme des eigentlichen Kopfanschlags 108 aufweist. Der Kopfanschlag 108 ist schwenkbar an einem Stift 166 gelagert, welcher in den Seitenwänden des Vorsprungs 164 verankert ist.

An der der Standsäule 102 zugewandten Seite des Vorsprungs 164 ist eine Federzunge 167 mit einem Rastnocken 168 angeformt. Der Kopfanschlag 108 weit eine zum Rastnocken 168 formkomplementäre Rastnut 169 auf, in welche der Rastnocken 168 einrasten kann, wenn sich der Kopfanschlag 108 in einer waagerechten Schwenkposition befindet. Dadurch wird der Kopfanschlag in der waagerechten Stellung arretiert. Zum Lösen der Arretierung muss der Kopfanschlag 108 nach unten gedrückt werden, so dass der Rastnocken 168 aus der Rastnut 169 gedrückt wird.

Der Schlitten 106 und der Kopfanschlag 108 sind so ausgeführt, dass sie ein von oben kommendes Messsignal möglichst wenig reflektieren, wenn sich der Kopfhebel in einer senkrechten Schwenkposition befindet. Dazu weist der Schlitten an der oberen Innenseite eine Anfasung 170 auf, so dass der Schlitten keine waagerecht verlaufenden Reflexionsflächen bietet. Der Kopfanschlag 108 ist in der senkrechten Schwenkposition nahezu vollständig von dem Vorsprung 164 verdeckt, so dass auch hier keine waagerechten Reflexionsflächen vorliegen.

Nur wenn der Kopfanschlag 108 sich in der waagerechten Schwenkposition befindet, kann er an seiner oberen Kante ein Messsignal reflektieren.

Am Kopfanschlag 108 kann ein Lagesensor 171 angeordnet sein, welcher der hier nicht dargestellten Steuerung meldet, ob sich der Kopfanschlag in einer waagerechten oder einer senkrechten Schwenkposition befindet. Auf Basis des Signals von dem Lagesensor 171, einer genauen Auswertung des reflektierten Messsignals, oder auf Basis einer manuellen Eingabe über die Eingabevorrichtung entscheidet die Steuerung 105, ob bei der Bestimmung der Körpergröße des Patienten 104, 104' die Dicke des Kopfanschlags 108 zu berücksichtigen ist.

## Patentansprüche

1. Messeinrichtung zur Bestimmung der Körpergröße eines Patienten (104,104'), mit einer an einer Standsäule (102) befestigten berührungslosen Messanordnung (103), wobei die Messanordnung (103) eingerichtet ist, ein Messsignal in Richtung des Kopfes eines unter der Messanordnung (103) stehenden Patienten (104,104') auszusenden, das reflektierte Messsignal aufzufangen und aus dem aufgefangenen Messsignal die Entfernung des Kopfes des Patienten (104,104') von der Messanordnung (103) zu bestimmen, und mit einer Steuerung (105), die eingerichtet ist, aus der so bestimmten Entfernung und der bekannten Position der Messanordnung (103) die Körpergröße des Patienten (104,104') zu bestimmen, wobei die Messeinrichtung weiterhin einen entlang der Standsäule (102) verfahrbaren Kopfanschlag (108) aufweist, **dadurch gekennzeichnet, dass** dem Kopfanschlag (108) ein Sensor (171) zur Bestimmung der Schwenkposition des Kopfanschlages (108) zugeordnet ist, und dass die Messanordnung (103) und/oder die Steuerung (105) eingerichtet ist, anhand der so bestimmten Schwenkposition des Kopfanschlages (108) zu erkennen, ob eine berührungslose Messung oder eine Messung mittels des Kopfanschlages (108) durchgeführt wird, wobei die Steuerung (105) eingerichtet ist, eine Dicke des Kopfanschlages (108) bei der Bestimmung der Körpergröße des Patienten (104) zu berücksichtigen.

2. Messeinrichtung nach Anspruch 1, **dadurch gekennzeichnet, dass** der Kopfanschlag (108) eingerichtet ist, ein von der Messanordnung (103) ausgesendetes Messsignal zu reflektieren.

3. Messeinrichtung nach Anspruch 1, **dadurch gekennzeichnet, dass** der Kopfanschlag (108) derart verschwenkbar an der Standsäule (102) angeordnet ist, dass er in einer ersten Schwenkposition ein von der Messanordnung (103) ausgesendetes Messsignal reflektiert, und dass er in einer zweiten Schwenkposition ein von der Messanordnung (103) ausgesendetes Messsignal nicht oder nur in einem sehr geringen Ausmaß reflektiert.

4. Messeinrichtung nach Anspruch 3, **dadurch gekennzeichnet, dass** der Kopfanschlag (108) an einem einen entlang der Standsäule (102) verfahrbaren Schlitten (106) verschwenkbar angeordnet ist.

5. Messeinrichtung nach Anspruch 3 oder 4, **dadurch gekennzeichnet, dass** der Kopfanschlag (108) in der ersten Schwenkposition arretiertbar ausgeführt ist.

6. Messeinrichtung nach einem der vorangehenden Ansprüche, **dadurch gekennzeichnet, dass** der Kopfanschlag (108) in einer Verfahrposition entlang der Standsäule (102) arretierbar ausgeführt ist.

7. Messeinrichtung nach einem der Ansprüche 1 bis 6, **dadurch gekennzeichnet, dass** die Messeinrichtung eine Eingabevorrichtung umfasst, und dass die Messanordnung (103) und/oder die Steuerung (105) eingerichtet ist, anhand einer Eingabe durch einen Benutzer zu entscheiden, ob eine berührungslose Messung oder eine Messung mittels des Kopfanschlages (108) durchgeführt werden soll.

8. Messverfahren zur Bestimmung der Körpergröße eines Patienten (104, 104'), mit den Schritten:
Positionieren eines Patienten (104,104') unter einer berührungslosen Messanordnung nach Anspruch 1 (103), wahlweise
Positionieren eines Kopfanschlags (108) an der Oberseite des Kopfes des Patienten (104') im Erfassungsbereich der Messanordnung (103), oder
Entfernen des Kopfanschlags (108) aus dem Erfassungsbereich der Messanordnung (103),
und
Bestimmen der Körpergröße des Patienten (104,104') mittels der Messanordnung (103),
wobei die Dicke des Kopfanschlags (108) bei der Bestimmung der Körpergröße des Patienten (104,104') berücksichtigt wird, wenn der Kopfanschlag im Erfassungsbereich der Messanordnung (103) positioniert ist und wobei dem Kopfanschlag (108) ein Sensor (171) zur Bestimmung der Schwenkposition des Kopfanschlages (108) zugeordnet ist, und wobei die Messanordnung (103) und/oder die Steuerung (105) eingerichtet ist, anhand der so bestimmten Schwenkposition des Kopfanschlages (108) zu erkennen, ob eine berührungslose Messung oder eine Messung mittels des Kopfanschlages (108) durchgeführt wird.

## Claims

1. Measurement apparatus for determining the height of a patient (104, 104'), having a contactless measurement device (103) secured on a stand (102), the measurement device (103) being designed to emit a measurement signal in the direction of the head of a patient (104, 104') standing under the measurement device (103), to capture the reflected measurement signal and to determine, from the captured measurement signal, the distance of the head of the patient (104, 104') from the measurement device (103), and having a controller (105) which is designed to determine the height of the patient (104, 104') from the distance thus determined and from the known position of the measurement device (103), the measurement apparatus moreover having a headpiece (108) movable along the stand (102), **characterized in that** the headpiece (108) is assigned a sensor (171) for determining the pivoting position of the headpiece (108), and **in that** the measurement device (103) and/or the controller (105) are/is designed to detect, on the basis of the thus determined pivoting position of the headpiece (108), whether a contactless measurement or a measurement by means of the headpiece (108) is performed, the controller (105) being designed to take into account a thickness of the headpiece (108) in the determination of the height of the patient (104).

2. Measurement apparatus according to Claim 1, **characterized in that** the headpiece (108) is designed to reflect a measurement signal emitted by the measurement device (103).

3. Measurement apparatus according to Claim 1, **characterised in that** the headpiece (108) is arranged pivotably on the stand (102) in such a way that, in a first pivoting position, it reflects a measurement signal emitted by the measurement device (103), and, in a second pivoting position, it does not reflect, or reflects only to a very small extent, a measurement signal emitted by the measurement device (103).

4. Measurement apparatus according to Claim 3, **characterized in that** the headpiece (108) is arranged pivotably on a carriage (106) that is movable along the stand (102).

5. Measurement apparatus according to Claim 3 or 4, **characterized in that** the headpiece (108) is designed to be lockable in the first pivoting position.

6. Measurement apparatus according to one of the preceding claims, **characterized in that** the headpiece (108) is designed to be lockable in a position of movement along the stand (102).

7. Measurement apparatus according to one of Claims 1 to 6, **characterized in that** the measurement apparatus comprises an input device, and **in that** the measurement device (103) and/or the controller (105) are/is configured to decide, on the basis of an input by a user, whether a contactless measurement or a measurement by means of the headpiece (108) is to be performed.

8. Measurement method for determining the height of a patient (104, 104'), with the following steps:
positioning a patient (104, 104') under a contactless measurement device (103) according to Claim 1,
or
positioning a headpiece (108) on the top of the head of the patient (104') in the detection range of the measurement device (103), or
removing the headpiece (108) from the detection range of the measurement device (103),
and
determining the height of the patient (104, 104') by means of the measurement device (103),
wherein the thickness of the headpiece (108) is taken into account in determining the height of the patient (104, 104') if the headpiece is positioned in the detection range of the measurement device (103), and wherein the headpiece (108) is assigned a sensor (171) for determining the pivoting position of the headpiece (108), and wherein the measurement device (103) and/or the controller (105) are/is designed to detect, on the basis of the thus determined pivoting position of the headpiece (108), whether a contactless measurement or a measurement by means of the headpiece (108) is performed.

## Revendications

1. Dispositif de mesure pour déterminer la taille d'un patient (104, 104'), comprenant un ensemble de mesure sans contact (103) fixé à une colonne (102), l'ensemble de mesure (103) étant aménagé pour émettre un signal de mesure en direction de la tête d'un patient (104, 104') debout sous l'ensemble de mesure (103), pour capter le signal de mesure réfléchi et pour déterminer la distance de la tête du patient (104, 104') par rapport à l'ensemble de mesure (103) à partir du signal de mesure capté, et comprenant un système de commande (105) qui est aménagé pour déterminer la taille du patient (104, 104') à partir de la distance ainsi déterminée et de la position connue de l'ensemble de mesure (103), le dispositif de mesure présentant en outre une butée de tête (108) déplaçable le long de la colonne (102), **caractérisé en ce qu'**un capteur (171) pour déterminer la position de pivotement de la butée de tête (108) est associé à la butée de tête (108), et **en ce que** l'ensemble de mesure (103) et/ou le système de commande (105) sont aménagés pour reconnaître à l'aide de la position de pivotement ainsi déterminée de la butée de tête (108) si une mesure sans contact ou une mesure au moyen de la butée de tête (108) est effectuée, le système de commande (105) étant aménagé pour tenir compte d'une épaisseur de la butée de tête (108) lors de la détermination de la taille du patient (104) .

2. Dispositif de mesure selon la revendication 1, **caractérisé en ce que** la butée de tête (108) est aménagée pour réfléchir un signal de mesure émis par l'ensemble de mesure (103).

3. Dispositif de mesure selon la revendication 1, **caractérisé en ce que** la butée de tête (108) est disposée pivotante sur la colonne (102) de telle sorte que dans une première position de pivotement, elle réfléchit un signal de mesure émis par l'ensemble de mesure (103), et **en ce que** dans une deuxième position de pivotement, elle ne réfléchit pas ou seulement très faiblement un signal de mesure émis par l'ensemble de mesure (103).

4. Dispositif de mesure selon la revendication 3, **caractérisé en ce que** la butée de tête (108) est disposée pivotante sur un chariot (106) déplaçable le long de la colonne (102).

5. Dispositif de mesure selon la revendication 3 ou 4, **caractérisé en ce que** la butée de tête (108) est réalisée de manière à pouvoir être arrêtée dans la première position de pivotement.

6. Dispositif de mesure selon l'une quelconque des revendications précédentes, **caractérisé en ce que** la butée de tête (108) est réalisée de manière à pouvoir être arrêtée dans une position de déplacement le long de la colonne (102).

7. Dispositif de mesure selon l'une quelconque des revendications 1 à 6, **caractérisé en ce que** le dispositif de mesure comprend un dispositif de saisie, et **en ce que** l'ensemble de mesure (103) et/ou le système de commande (105) sont aménagés pour décider à l'aide d'une saisie par un utilisateur si une mesure sans contact ou une mesure au moyen de la butée de tête (108) doit être effectuée.

8. Procédé de mesure pour déterminer la taille d'un patient (104, 104'), comprenant les étapes consistant à :
positionner un patient (104, 104') sous un ensemble de mesure sans contact (103) selon la revendication 1,
en option
positionner une butée de tête (108) au niveau du sommet de la tête du patient (104') dans la zone de détection de l'ensemble de mesure (103), ou
retirer la butée de tête (108) de la zone de détection de l'ensemble de mesure (103),
et
déterminer la taille du patient (104, 104') au moyen de l'ensemble de mesure (103),
dans lequel l'épaisseur de la butée de tête (108) est prise en compte lors de la détermination de la taille du patient (104, 104') si la butée de tête est positionnée dans la zone de détection de l'ensemble de mesure (103), et dans lequel un capteur (171) pour la détermination de la position de pivotement de la butée de tête (108) est associé à la butée de tête (108), et dans lequel l'ensemble de mesure (103) et/ou le système de commande (105) sont aménagés pour reconnaître à l'aide de la position de pivotement ainsi déterminée de la butée de tête (108) si une mesure sans contact ou une mesure au moyen de la butée de tête (108) est effectuée.
